# EUROPEAN PATENT APPLICATION

(11) **EP 4 287 199 A1**
(43) Date of publication of application: **06.12.2023**
(21) Application number: 22176636.3
(22) Date of filing: 01.06.2022
(51) Int. Cl.: G16H 30/20, G16H 30/40, A61B 6/14, G16H 10/60, A61B 6/00, A61B 5/00

(54) **DENTAL USER INTERFACES**

(71) Applicant: Sirona Dental Systems GmbH, 64625 Bensheim (DE); DENTSPLY SIRONA Inc., York, PA 17401 (US)
(72) Inventor: Tim, Braun, 64625 Bensheim (DE)
(74) Representative: Özer, Alpdeniz

(57) **Abstract**

The present teachings relate to a method for improving usability of dental imaging data, comprising: providing imaging data comprising a plurality of different images of at least one dental imaging modality of a patient; providing metadata for each of the images; selecting one or some of the images; retrieving in response to the selection at least one another image; wherein the retrieval is performed by comparing metadata of at least one of the selected images and metadata of at least one of the retrieved images. The present teachings also relate to systems, software products and storage media.

## Description

### TECHNICAL FIELD

The present teachings relate generally to computer-implemented methods and products for dental imaging.

### BACKGROUND

Modern day dentistry is evolving in the digital domain. Output of most diagnosis systems in the market is already in digital form, but now there is a trend of leveraging data-driven tools for capturing insights and making the work of the dental practitioner as easy as possible. Data-driven techniques rely upon availability of huge amounts of data. Data from multiple sources are collected for analysis via data-driven logics and/or for use as training data. This trend is enabled not only by steady reduction of costs of processing power, but also in reduction of costs of storing data. Accordingly, ever increasing amounts of data, or the so-called bigdata, are being saved and archived. These data may be hugely beneficial for leveraging data-driven techniques.

There are also other aspects to it, for example, large amounts of data may remain underutilized. The problem may be further aggravated by the fact that it may be difficult to know what exactly is available. For example, there may be large number of images available from different scans which are saved in a database, however, it may require a pre-knowledge that they are available to even know if to look for them. Even if known, it may be a challenge to find those from the huge amount of data which are stored.

The applicant has realized that there is a need for improved methods and products which can better assist a dental health practitioner by leveraging data more efficiently.

### SUMMARY

At least some of the limitations associated with the foregoing can be overcome by the subject matter of the accompanying independent claims. At least some of the additional advantageous alternatives will be outlined in the dependent claims.

When viewed from a first perspective, there can be provided a computer-implemented method for improving usability of dental imaging data, which method comprises:
- providing imaging data comprising a plurality of different images of at least one dental imaging modality of a patient;
- providing metadata for each of the images; wherein the metadata related to a specific image comprise semantic information related to one or more anatomical features recorded in that image,
- selecting, from the imaging data, one or some of the images;
- retrieving in response to the selection, from the imaging data, at least one another image; wherein the retrieval is performed by comparing metadata of at least one of the selected images and metadata of at least one of the retrieved images.

The applicant has realized that by doing so, the imaging data related to at least one different image can be automatically retrieved, without the user having to find out what is available in the provided imaging data. The present teachings achieve this by establishing a link between specific images in the imaging data. These links are leveraged to retrieve the at least one another image, or retrieved imaging data, based on the selected one or some images, or the selected imaging data. More specifically, this link is advantageously established using semantic information related to anatomical features in the different images in the imaging data. Thus, the present teachings establish and maintain semantic correspondence between the different images in the imaging data, irrespective of the number of imaging modalities the imaging data may have.

For ease of understanding and without limiting the scope or generality of the present teachings, the terms "selected imaging data", "selected one or some images" or "selected image" shall be used interchangeably in the present disclosure. Similarly, the terms "retrieved imaging data", "retrieved image", or "at least one another image may be used interchangeably in the present disclosure.

Thus, the semantic information can be correlated to select the imaging data and/or retrieve the imaging data related to at least one another image. Different realizations of the teachings will be discussed in the present disclosure along with their technical advantages. The selected imaging data are a subset, i.e., at least one image, of the provided imaging data. Similarly, the retrieved imaging data are another subset of the provided imaging data, i.e., at least one another image different from the selected image, which is/are retrieved in response to the comparison of the metadata.

"Metadata" of a specific image, or of a specific part of the imaging data, refers to data which comprise information about the image or the part of the imaging data. More specifically, the metadata of an image comprises semantic information which is related to the anatomical features of that image.

"Semantic information" in the present context refers to interpreted or inferenced data related to one or more anatomical features. When related to a specific part of imaging data, e.g., an image, the semantic information may be interpretation of what can be observed or found from the image. The semantic information is related to one or more anatomical features. As a few non-limiting examples, the semantic information may comprise any one or more of the anatomical features: a specific tooth number or reference, a specific nerve, a specific bone, jaw type, a specific pathology, a specific replacement, a specific treatment or procedure, etc. Advantageously, the semantic information also includes information of the location where the tooth can be found in the image. For example, the semantic information may include information of most or all pixels or voxels of the image which are related to a specific tooth. Hence, using the semantic information, that specific tooth may be highlighted or enclosed with an outline. Alternatively, or additionally, the semantic information may provide a general location of that tooth. Hence, using the semantic information, that specific tooth may be pointed to, e.g., by placing a point or tag at or around the middle of the tooth.

In many cases, after acquisition of a scan, such as an X-ray image, a dental practitioner tags or marks the image with their interpretation or observations. These data can be used as semantic information. Alternatively, or additionally, the semantic information may be automatically provided by computationally processing the imaging data, completely or partially. The processing may advantageously be using one or more data-driven logics which are provided the imaging data as an input, and the logic(s) provide semantic information for the imaging data. This semantic information may either be used directly as the metadata, or it may be processed further to provide the metadata for each of the images processed via the logic(s). The logic(s) may perform segmentation and/or localization operations on each of the images to provide semantic information for the respective image.

Preferably, the semantic information is organized in different abstraction categories. Thus, imaging data may provide the semantic information, or parts thereof, in different categories, levels or grades of abstraction. Also preferably, each or some parts of semantic information may contain relationship information to other parts, thus forming a network of related information across various abstraction levels. For example, some semantic information with a low level of abstraction may comprise information such as location of a specific anatomical feature, such as location of a specific implant, abutment, tooth, nerve, etc., whereas at a higher level of abstracted information may be a category layer which comprises information which describe entities with dental terminology such as teeth number or type, gingiva, bone, prosthetic, maxillary, etc. There may even be a pathology level abstraction and/or procedure level abstraction which may link information at various abstraction levels. As a few non-limiting examples, pathology level may contain information such as caries, fractures, osseointegration, etc. which may establish link e.g., to specific teeth at different locations in the oral anatomy. Similarly, procedure level may contain information such as osteotomy, endodontic treatment, etc. which may be linked to specific parts of the pathology level and/or lower abstraction levels. There may even be other levels, below, above, at the same level, or between any of the levels or categories discussed above.

An advantage of arranging the semantic information is that the comparison between the metadata of the selected imaging data and their matching other imaging data can be improved for better retrieval of relevant data. Hence, the availability and usability of the imaging data are improved for the user even if there is not a direct match between which image the user selected from the imaging data and the retrieved images. In bigdata applications, it can be highly challenging for the user to even realize what is available, let alone searchable. Hence, semantic correspondences between the images in the imaging data are leveraged to extract relevant images even without the user knowing or realizing about their existence.

Another advantage can be that even if there is not complete match, but the linked path between the levels or layers is same or similar, the retrieval can still be performed. For example, when a selection is made based on endodontic treatment and maxillary side, those image data which are associated with endodontic treatment on the specific tooth may still be retrieved as the semantic information provided can still be associated with the maxillary side. In other cases, the semantic information can be used to filter data which are not relevant. Taking the same example, when a selection is made based on endodontic treatment and maxillary side, only the image data associated with the tooth in question post-treatment may be retrieved. As there may be other images of the tooth which are pre-treatment or even pre-pathology, these images may not be retrieved to prevent irrelevant data from being provided.

"Anatomical feature" refers to information related to a specific anatomy of the patient, more specifically the craniofacial anatomy of the patient. For example, an anatomical feature may be information related to a specific tooth or a group of teeth. Thus, anatomical features may even refer to information related to any one or more intraoral structures such as, dentition, gingiva, nerve channel, extraction site, jawbone, and condyle. Alternatively, or in addition, anatomical features may be one or more artificial structures such as one or more dental replacements, e.g., dental crown, braces, veneer, bridge. Alternatively, or in addition, an anatomical feature may in some cases even be a scan body (or scanbody) or other natural and/or artificial structure attached to the jaw of the patient. Alternatively, or additionally, the anatomical feature may even be information related to a pathology or condition. As non-limiting examples, pathology or condition may be any one or more of, fracture of tooth or bone, caries, radiolucency, impaction, missing tooth, or any other characterizable state of the oral anatomy or any part thereof.

"Imaging data" or "image data" in the present context refers to data which at least partially have been obtained from an imaging of a patient. The imaging data comprise a plurality of different images of at least one dental imaging modality of the same patient. As a few non-limiting examples, the dental imaging modality may be X-ray, Orthopantomogram ("OPG"), cephalogram ("CEPH"), cone-beam computed tomography ("CBCT"), optical oral photography, optical 3D surface scan, or their likes. The images included in the imaging data may be of the same type, and/or preferably of different types. As non-limiting examples, the image may be an X-ray sinogram, a DVT image, a panoramic image, a bitewing image, a CEPH image, a photographic image such as a photographic dental arch image, a radiographic projection image or their likes of the patient.

The step of selecting imaging data related to one or more of the images may occur in response to a user input, or it may be automatically performed in response to a preceding process. Thus, according to an aspect, the selection of imaging data is performed in response to a user input. For example, the user input may provide one or more diagnostic parameters. The selection is thus performed by matching any one or more of the diagnostic parameters with the metadata of the images in the imaging data. The diagnostic parameter may be a reference to one or more anatomical features. Alternatively, or additionally, any of the diagnostic parameters may be based on any one or more or: workflow, direct text, treatment, pathology, or their likes. As a non-limiting example, the one or more diagnostic parameters may relate to progression of periodontal bone loss. The user may select or provide input, for example a text string *periodontal bone loss.*

Thus, pursuant to the present teachings, those imaging data which relate to the patient's images (such as X-ray and digital impressions) depicting the bone crest in the region of interest are automatically retrieved by comparing the metadata. The dental practitioner is hence enabled to perform a more thorough diagnosis without spending additional effort in knowing if other images exist, or even if known searching for them manually.

More advantageously, the selected and retrieved imaging data are presented in a display arrangement comprising image views in a manner which makes it easier for the user to make the relevant diagnosis. For example, the image views may be arranged automatically in chronological order (e.g., date of capture). Further advantageously, the view of each image is automatically adapted using the semantic information such that the user is assisted in making a diagnosis. For example, orientation and/or magnification of each or some of the images may be adjusted such that each view presents a comparable view of the bone crest, which eases comparison of bone crest height at different time points.

In the present context, an image view refers to a single image selected or retrieved from the imaging data, and is adapted with additional properties defining especially the visible part of the image shown on the HMI, for example any one or more of: parameterized by level of magnification, 3D orientation and center of the displayed data, or any other configurations such as the mapping of data value to displayed brightness or view interpolation. The additional properties are applied in response to the semantic data of the image and/or the comparison of the metadata. In some cases, the additional properties of one of the image views may be provided via a user input. Consequently, pursuant to the present teachings, the corresponding additional properties are applied automatically to at least some of the other image views in the display arrangement. It is also possible that some of the image views are deleted and/or additional image views are added to the display arrangement by retrieving additional imaging data as proposed. The additional image views are also adapted by applying corresponding additional properties as proposed.

Thus, the retrieved imaging data are preferably included in the display arrangement together with the selected imaging data for the user as a plurality of image views. For example, based on the selection of the imaging data, which may be automatic or based on a specific user input, a specific image view of the imaging data may be provided at a user interface or human machine interface ("HMI"). The image view may for example be a view of a certain part of the oral anatomy selected from one or more different imaging modalities. Preferably, the image view is configured with one or more additional properties based on the comparison and/or the semantic information. Thus, the image view may be formed from the retrieved imaging data set and preferably configured with additional properties such as the displayed part of the data.

The display arrangement is automatically configured to support the user's diagnostic task or interest, based on the semantic data and/or the comparison.

In contrast to classical image registration methods, which establish spatial correspondences between images based on locally similar brightness or color distributions, the proposed usage of semantic information works at a higher level of abstraction. Thus, the present teachings can enable to relate representations of the anatomical features which are present in visually very different modalities. Changed position / orientation of a patient, non-rigid movements (open / closed mouth position) and distortions due to the used (projective) imaging technique in different images may also be compensated by the present teachings, which is a distinct advantage over classical image registration approaches when configuring image views to show similar semantic content.

Thus, according to an aspect, at least a part of the selected imaging data and at least a part of the retrieved imaging data may be provided at an interface, such as an HMI and/or an application programming interface ("API"). Based upon the application the interface may be a software and/or a hardware interface. The advantage of providing said data to an HMI is that the user is displayed the relevant data automatically by establishing and maintaining semantic correspondence between the selected imaging data and the retrieved imaging data. Thus, the retrieved imaging data are interactively adapted accordingly. Such a change may be in response to a user input on any of the imaging data being shown in the HMI. Hence, the user is continuously automatically provided data which can be useful for reaching the correct diagnosis or conclusion. This can also help in discovering underlying health conditions earlier. Thus, the method may further comprise:
- updating the part of the selected imaging data;
- removing, in response to the update, at least a part of the retrieved imaging data; and/or
- retrieving, in response to the update, another different imaging data.

Thus, as the selected imaging data change, the retrieved imaging data are adapted accordingly. Hence, the part of the retrieved imaging data at the interface may also be updated.

According to an aspect, the method may further comprise:
- updating a part of the display arrangement, or an image view in the display arrangement;
- removing, in response to the update, at least one of the image views; and/or
- retrieving, in response to the update, at least one yet another image from the imaging data to provide at least one new image view in the display arrangement.

Thus, when the display arrangement is updated, e.g., one of the image views in the arrangement is manipulated by scrolling, zoom, or pan, etc., then in response to the update one or more of the imaging views may be automatically removed from the arrangement. Additionally, or alternatively, one of more different or additional image views may be added or included in the arrangement by performing a retrieval step as was earlier proposed. Hence, the removal and the retrieval as appropriate are performed in response to the comparison and/or the semantic data. As it was shown in the bone loss example above, the diagnostic parameter can be leveraged to configure the retrieved imaging data which are provided to the interface. For example, the magnification and/or perspective view and/or temporal fashion in which the imaging data are arranged can be determined in response to any one or more of the diagnostic parameters. Thus, further advantageously, the display arrangement may be automatically configured dependent upon any one or more of the diagnostic parameters and/or the metadata of retrieved images.

As it was discussed, the metadata may be based on annotations, which may be user provided and/or automatically provided. Thus, according to an aspect, at least a part of the metadata is provided via a data-driven logic. The data-driven logic may perform semantic processing on the imaging data for providing at least a part of the metadata.

"Data-driven logic" refers to refers to a logic, which at least partially derives its functionality from training data. In contrast to a rigorous or analytical logic which is based on programmed instructions for performing a particular logical task, a data-driven logic can allow forming such instructions at least partially automatically using the training data. The use of data-driven logic can allow to describe logical and/or mathematical relationships without explicit manual algorithm design. This can reduce computational requirements and/or improve speed. Moreover, a data-driven logic may be able to detect patterns or indications (e.g., in input or input data provided to the data-driven logic) which can otherwise not be known or may be difficult to implement as an analytical logic form.

The data-driven logic may be in software and/or hardware form, for example, executable via one or more computing units.

It shall be appreciated that in the present context, the data-driven logic refers to a trained mathematical logic which is parametrized according to the respective training data set. For example, the anatomy data-driven logic is parameterized via its respective training data to detect one or more anatomical features. An untrained logic lacks this information. Hence, the untrained logic or model is incapable of performing a desired detection. Feature engineering and training with the respective training datasets thus enables parametrization of the untrained logic. The result of such a training phase is the respective data-driven model, which preferably solely as a result of the training process, provides interrelations and logical capability related to the purpose for which the respective data-driven logic is to be used. In this case, the data-driven logic may be trained with a training dataset comprising a plurality of images and their corresponding semantic information.

When the data-driven logic is used for inferencing, the imaging data is provided at its input and the data-driven logic provides at least a part of the metadata as output.

Any of the imaging data and/or metadata may be provided at the same memory storage or different ones.

When viewed from another perspective, there can also be provided a system comprising means for performing the steps of any of the methods herein disclosed.

For example, there can be provided a system for improving usability of dental imaging data, the system comprising one or more computing units, wherein any of the computing units is configured to:
- provide imaging data comprising a plurality of different images of at least one dental imaging modality of a patient;
- provide metadata for each of the images; wherein the metadata related to a specific image comprise semantic information related to one or more anatomical features recorded in that image,
- select, from the imaging data, one or some of the images;
- retrieve, in response to the selection, from the imaging data at least one another image; wherein the retrieval is performed by comparing metadata of at least one of the selected images and metadata of at least one of the retrieved images.

The system may be an add-on to a diagnosis system such as an imaging system, or it may be a part of it. In some cases, the system may at least partially be implemented as a cloud-computing service. When implemented as an add-on, the diagnosis system and the proposed system may be interconnected via a connectivity interface and/or a network interface. Any two or more of the connectivity interface and/or the network interface and/or the interface may be the same device or they may be different ones.

When viewed from yet another perspective, there can also be provided a computer software product, or a non-transitory computer-readable storage medium storing the program, comprising instructions which when executed by one or more suitable computing units cause any of the computing units to perform the steps of any of the methods herein disclosed.

For example, there can be provided a computer software product, or a non-transitory computer-readable storage medium storing the program, comprising instructions which when executed by one or more suitable computing units cause any of the computing units to:
- provide imaging data comprising a plurality of different images of at least one dental imaging modality of a patient;
- provide metadata for each of the images; wherein the metadata related to a specific image comprise semantic information related to one or more anatomical features recorded in that image,
- select, from the imaging data, one or some of the images;
- retrieve, in response to the selection, from the imaging data at least one another image; wherein the retrieval is performed by comparing metadata of at least one of the selected images and metadata of at least one of the retrieved images.

"Computing unit", "computing device", "processing unit" or "processing device" may comprise, or it may be, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"), the processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Network interface" refers to a device or a group of one or more hardware and/or software components that allow an operative connection with the network.

"Connectivity interface" or "communication interface" refers to a software and/or hardware interface for establishing communication such as transfer or exchange of signals or data. The communication may either be wired, or it may be wireless. Connectivity interface is preferably based on or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long-distance protocol. The connectivity interface may support any one or more standards and/or proprietary protocols.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a graphical aspect of the present teachings;
FIG. 2 illustrates another graphical aspect of the present teachings;
FIG. 3 illustrates an aspect for providing the metadata pursuant to the present teachings;
FIG. 4 illustrates flowchart of an aspect of the present teachings.

### DETAILED DESCRIPTION

In accordance with example aspects described herein, methods, systems and computer readable storage media can be provided, e.g., for improving usability of dental imaging data.

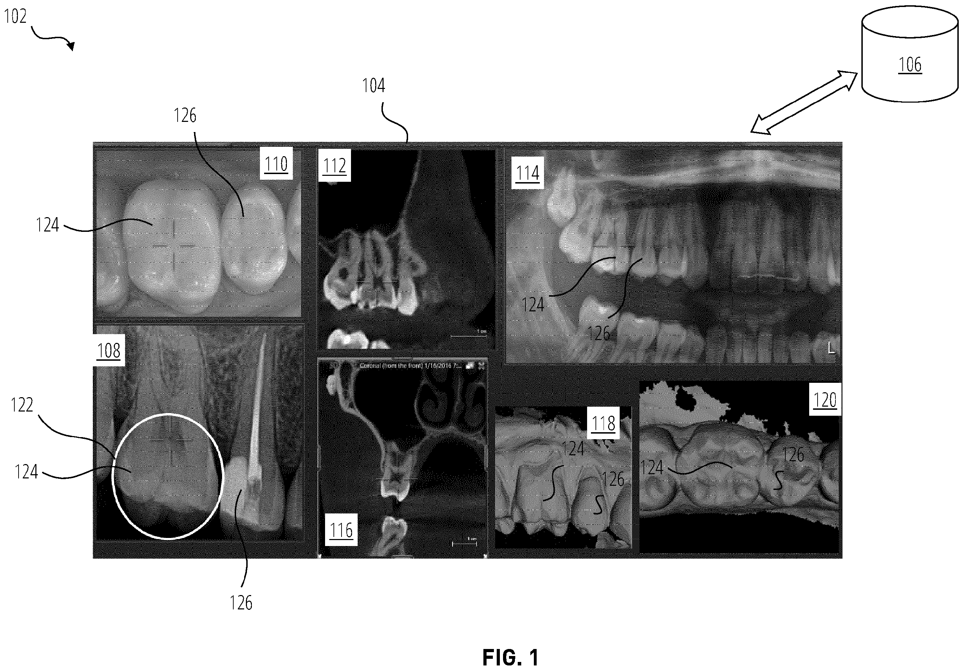

FIG. 1 shows a block diagram 102 demonstrating a graphical aspect of the present teachings. A display arrangement 104 is shown which comprises a plurality of dental image views 108 - 120. The dental image views 108 - 120 are image views of the imaging data which may be stored in a memory storage 106. The memory storage 106 may be a single unit or it may be distributed over different units located at the same or different geographical locations. The imaging data comprise a plurality of different images (including 108 - 120) of a patient. As can be seen in this example, the imaging data relates to different imaging modality of the patient. The display arrangement 104 may be provided to an interface such as an HMI or graphical user interface.

The display arrangement 104 in this example comprises a coronal slice through a CBCT volume 116, an intraoral optical image 110, an X-ray intraoral image 108, an X-ray panoramic image 114, a surface scan image 118 with a view from a given perspective or orientation, and another surface scan image 120 with a view from another perspective or orientation as compared to the surface scan image 118.

The intra-oral X-ray image 108 is shown in this example with a marker or circle 122. The circle 122 is placed to illustrate the common features which are shown by the different views 108, 110, 112, 114, 116, 118 and 120. The display arrangement 104 is thus automatically adapted pursuant to the present teachings to show relevant imaging data to a user such as a dental practitioner. For example, the display arrangement 104 may have been triggered by a selection made by the dental practitioner while viewing the image 108. Thus, the X-ray intraoral image 108 may represent a selected image or selected imaging data. In response to the selection, the another imaging data or the another images 110 - 120 are automatically retrieved. The retrieval is performed by comparing metadata of the X-ray intraoral image 108 and metadata of the other images in the imaging data. The result is that the retrieved images 110 - 120 are included in the display arrangement 104. As shown, the views 110 - 120 of the retrieved image data are adapted according to the relevance by using the semantic information and/or the comparison of the metadata. For example, it may be detected from the metadata or the semantic information which features or context are relevant for the displayed portion of the X-ray intraoral image 108, and based on that the semantic information of the other views 110 - 120 may be analyzed to adapt the displayed portion of each of the views 110 - 120. The user can thus be automatically provided most relevant imaging data.

The selected image view 108 is showing a molar 124, indicated by the marker 122. Other anatomical features such as a root-filled premolar 126 is also visible in the CBCT image view 108. By comparing the metadata of the X-ray intraoral image 108, the views of the retrieved images 110 - 120 have been automatically adapted accordingly for the practitioner to view features of the molar 124 in different forms. For example, the 110 is zoomed and panned to the molar 124, and so are the images 118 and 120 to show the molar 124 from different perspectives. The views and adaptations can further be influenced by one or more diagnostic parameters which may be provided by the dental practitioner.

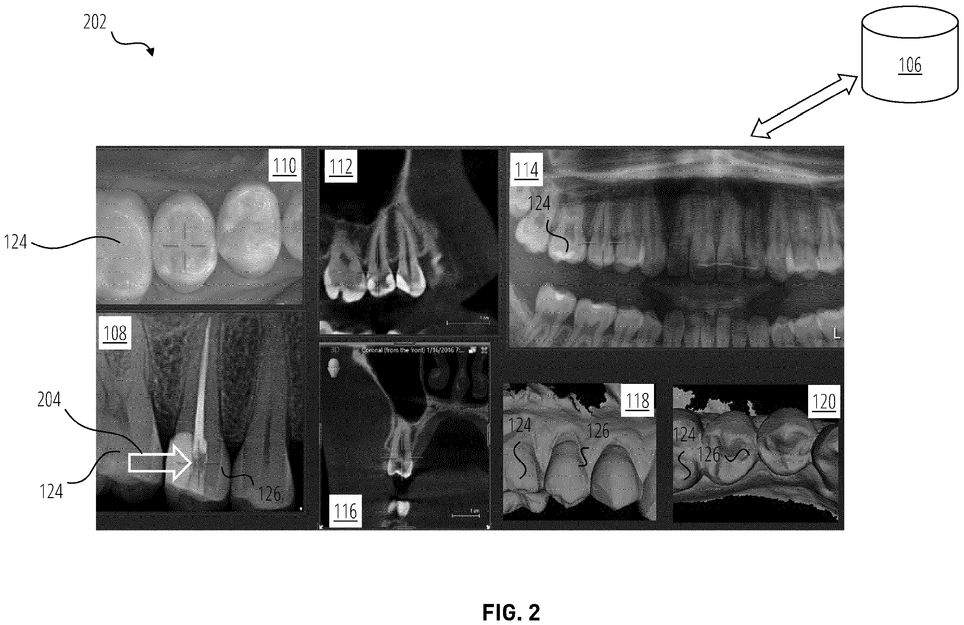

FIG. 2 shows another block diagram 202 of the display arrangement 104 in which the selection has been altered, e.g., by the user through interaction with the image 108 - in this example, the user may have panned the view 108 to the right of the image 108.

Hence, in this case, the dental practitioner has decided to focus on the root-filled premolar 126 instead of the molar 124. So, the panning operation performed by the user is depicted here as an arrow 204. Accordingly, each of the another views 110 - 120 have been adapted according to the updated graphical content visible in the image 108. In some cases, even other images in the imaging data may match with the metadata of the new selection of the X-ray intraoral image 108, in that case, such matching views or images may also be included in the display arrangement 104. Alternatively, if any of the other views 110 - 120 would not match the new selection, those views could be removed from the display arrangement 104.

The dental practitioner is thus interactively assisted in focusing on the relevant data, thus helping in providing a more accurate diagnosis and treatment.

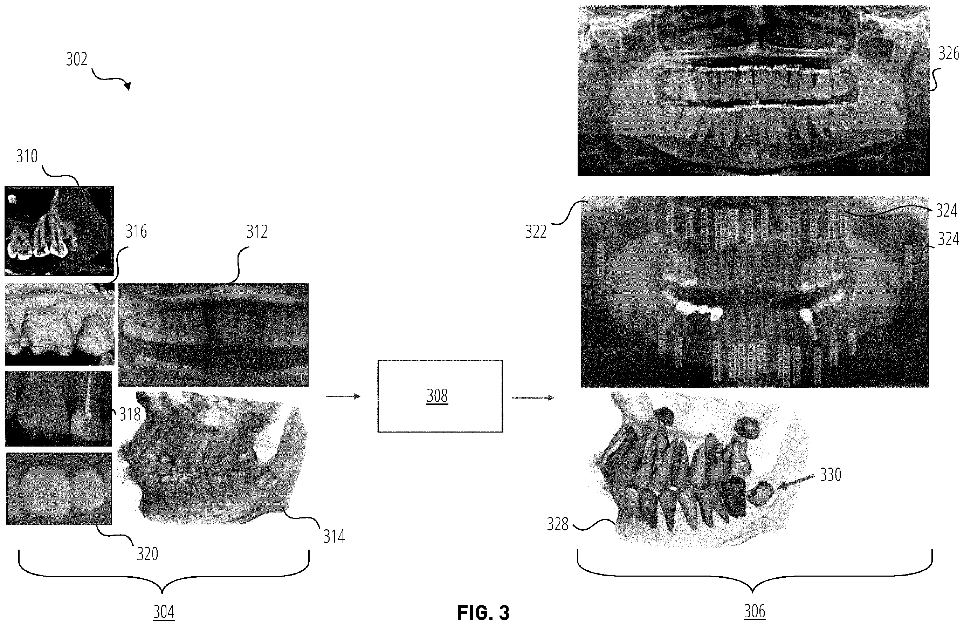

FIG. 3 shows a flow diagram 302 illustrating a way to provide metadata for the imaging data 304. The imaging data 304 is shown here comprising a plurality of dental views 310 - 320 of different imaging modalities. In this example, the imaging data 304 comprise a sagittal slice 310 of a CBCT volume, an X-ray intraoral image 318, a panoramic image 312, a 3D volume 314, a surface scan 316 and an intraoral optical image 320. The imaging data 304 are processed via an image analysis logic 308, which preferably is at least partially a data-driven logic. The image analysis logic 308 may perform an automated semantic analysis of the imaging data 304.

A result of the processing is that imaging data with metadata 306 comprising semantic information are provided. The metadata may or may not be saved together with the imaging data. Thus, the metadata may be saved at another memory storage than that storing the imaging data 304. The output of the processing may, for example, be a first annotated panoramic image 322 which comprises annotations 324. The annotations 324 may provide location of the respective anatomical features in the first annotated panoramic image 322 and/or it may segment the image according to the anatomical features present in the image 322. The metadata of an image comprise semantic information related to one or more anatomical features recorded in that image, for example, tooth number, root naming, view direction, etc. Thus, the metadata may comprise any one of more of: outline, voxel mask, semantic labels, and their likes.

Similar to the first annotated panoramic image 322, a second annotated panoramic image 326 and an annotated 3D volume 328 is also provided with their respective metadata comprising semantic information. For example, metadata of the annotated 3D volume 328 comprises a volume annotation 330, which may specify that specific voxels relate to an unerupted developing molar, with FDI 38, on the left labial side of the patient.

These metadata can thus be leveraged pursuant the present teachings. For example, in response to a user selection of an initial image, task or workflow.

On user interaction with any one of the images which are in the display arrangement 104, semantically consistent update can be provided for the entire display arrangement 104. For interactive continuous movements, a non-linear interpolation of intermediate display positions, e. g. using a 2D/3D thin-plate-spline as a displacement field may be computed from the semantic information of the imaging data. If optimal workflow requires a specific alteration of the 1-to-1 spatial correspondence, this can also be incorporated during transfer of interaction.

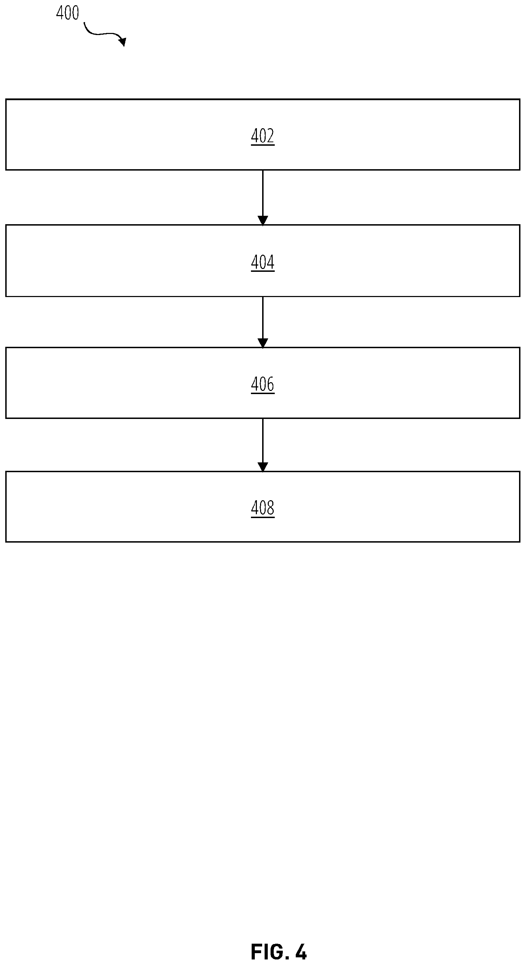

FIG. 4 shows a flowchart 400 exemplifying an aspect of the present teachings. The flowchart 400 can be implemented as a routine executable by one or more computing units, for example, operatively connected to a diagnosis system, for example, a dental imaging system.

In block 402, it is provided imaging data comprising a plurality of different images of at least one dental imaging modality of a patient.

In block 404, it is provided metadata for each of the images; wherein the metadata related to a specific image comprise semantic information related to one or more anatomical features recorded in that image.

In block 406, it is selected from the imaging data, one or some of the images. Thus, at least one, but not all images, are selected from the imaging data.

In block 408, it is retrieved, in response to the selection, from the imaging data at least one another image. The retrieval is performed by comparing metadata of at least one of the selected images and metadata of at least one of the retrieved images.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for improving usability of dental imaging data, comprising: providing imaging data comprising a plurality of different images of at least one dental imaging modality of a patient; providing metadata for each of the images; selecting one or some of the images; retrieving in response to the selection at least one another image; wherein the retrieval is performed by comparing metadata of at least one of the selected images and metadata of at least one of the retrieved images. The present teachings also relate to systems, software products and storage media. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect. Certain aspects of the present teachings are summarized in the following clauses.
Clause 1. A computer-implemented method for improving usability of dental imaging data, which method comprises:
   - providing imaging data comprising a plurality of different images of at least one dental imaging modality of a patient;
   - providing metadata for each of the images; wherein the metadata related to a specific image comprise semantic information related to one or more anatomical features recorded in that image,
   - selecting, from the imaging data, one or some of the images;
   - retrieving in response to the selection, from the imaging data, at least one another image; wherein the retrieval is performed by comparing metadata of at least one of the selected images and metadata of at least one of the retrieved images.
Clause 2. Method of clause 1, wherein the selection of the images is performed in response to a user input.
Clause 3. Method of clause 2, wherein the user input provides one or more diagnostic parameters, and the selection is performed by matching any one or more of the diagnostic parameters with the metadata.
Clause 4. Method of any one of clauses 1 to 3, wherein at least one of the selected images and at least one of the retrieved images are provided at an interface, such as a human machine interface in the form of a plurality of image views.
Clause 5. Method of clause 4, wherein the image views displayed at the human machine interface are arranged in a display arrangement dependent upon any one or more of the diagnostic parameters and/or the metadata of retrieved images.
Clause 6. Method of clause 5, further comprising:
   - updating a part of the display arrangement, or an image view in the display arrangement;
   - removing, in response to the update, at least one of the image views; and/or
   - retrieving, in response to the update, yet another image from the imaging data to provide at least one new image view in the display arrangement.
Clause 7. Method of any one of clauses 1 to 6, wherein at least a part of the metadata is provided via a data-driven logic.
Clause 8. Method of clause 7, wherein the data-driven logic performs semantic processing on the imaging data for providing the metadata.
Clause 9. Method of any one of clauses 1 to 8, wherein the semantic information comprises a hierarchical structure and/or parallel structure.
Clause 10. A system comprising means for performing the steps of any of the above method clauses.
Clause 11. A computer software product, or a non-transitory computer-readable storage medium storing the program, comprising instructions which when executed by one or more suitable computing units cause any of the computing units to perform the steps of any of the above method clauses.
Clause 12. A system for improving usability of dental imaging data, the system comprising one or more computing units, wherein any of the computing units is configured to:
   - provide imaging data comprising a plurality of different images of at least one dental imaging modality of a patient;
   - provide metadata for each of the images; wherein the metadata related to a specific image comprise semantic information related to one or more anatomical features recorded in that image,
   - select, from the imaging data, one or some of the images;
   - retrieve, in response to the selection, from the imaging data at least one another image; wherein the retrieval is performed by comparing metadata of at least one of the selected images and metadata of at least one of the retrieved images.
Clause 13. A computer software product, or a non-transitory computer-readable storage medium storing the program, comprising instructions which when executed by one or more suitable computing units cause any of the computing units to:
   - provide imaging data comprising a plurality of different images of at least one dental imaging modality of a patient;
   - provide metadata for each of the images; wherein the metadata related to a specific image comprise semantic information related to one or more anatomical features recorded in that image,
   - select, from the imaging data, one or some of the images;
   - retrieve, in response to the selection, from the imaging data at least one another image; wherein the retrieval is performed by comparing metadata of at least one of the selected images and metadata of at least one of the retrieved images.

## Claims

1. A computer-implemented method for improving usability of dental imaging data, which method comprises:
- providing imaging data comprising a plurality of different images of at least one dental imaging modality of a patient;
- providing metadata for each of the images; wherein the metadata related to a specific image comprise semantic information related to one or more anatomical features recorded in that image,
- selecting, from the imaging data, one or some of the images;
- retrieving in response to the selection, from the imaging data, at least one another image; wherein the retrieval is performed by comparing metadata of at least one of the selected images and metadata of at least one of the retrieved images.

2. Method of claim 1, wherein the selection of the images is performed in response to a user input.

3. Method of claim 2, wherein the user input provides one or more diagnostic parameters, and the selection is performed by matching any one or more of the diagnostic parameters with the metadata.

4. Method of any one of claims 1 to 3, wherein at least one of the selected images and at least one of the retrieved images are provided at an interface, such as a human machine interface in the form of a plurality of image views.

5. Method of claim 4, wherein the image views displayed at the human machine interface are arranged in a display arrangement dependent upon any one or more of the diagnostic parameters and/or the metadata of retrieved images.

6. Method of claim 5, further comprising:
- updating a part of the display arrangement, or an image view in the display arrangement;
- removing, in response to the update, at least one of the image views; and/or
- retrieving, in response to the update, yet another image from the imaging data to provide at least one new image view in the display arrangement.

7. Method of any one of claims 1 to 6, wherein at least a part of the metadata is provided via a data-driven logic.

8. Method of claim 7, wherein the data-driven logic performs semantic processing on the imaging data for providing the metadata.

9. Method of any one of claims 1 to 8, wherein the semantic information comprises a hierarchical structure and/or parallel structure.

10. A system comprising means for performing the steps of any of the above method claims.

11. A computer software product, or a non-transitory computer-readable storage medium storing the program, comprising instructions which when executed by one or more suitable computing units cause any of the computing units to perform the steps of any of the above method claims.
